# EUROPEAN PATENT APPLICATION

(11) **EP 1 764 102 A1**
(43) Date of publication of application: **21.03.2007**
(21) Application number: 05755839.7
(22) Date of filing: 01.07.2005
(51) Int. Cl.: A61K 31/4709, A61K 47/02

(54) **QUINOLONE-CONTAINING MEDICINAL COMPOSITION**

(30) Priority: 02.07.2004 JP 2004197223
(71) Applicant: DAIICHI PHARMACEUTICAL CO., LTD., Chuo-ku, Tokyo 103-8234 (JP)
(72) Inventor: YANO, Emi, Utsunomiya-shi, Tochigi 3210923 (JP); KOBAYASHI, Hideo, Daiichi Pharmaceutical Co., Ltd., Edogawa-ku, Tokyo 1348630 (JP); KIKUCHI, Hiroshi, Daiichi Pharmaceutical Co., Ltd., Edogawa-ku, Tokyo 1348630 (JP); YAMAGUCHI, Yuri, Daiichi Pharmaceutical Co., Ltd., Edogawa-ku, Tokyo 1348630 (JP); JINDO, Toshimasa, Daiichi Pharmaceutical Co., Ltd., Edogawa-ku, Tokyo 1348630 (JP); NISHIMOTO, N., Daiichi Pharmaceutical Co., Ltd., Takatsuki-shi, Osaka5 690806 (JP)
(74) Representative: Blodig, Wolfgang
(86) International application number: PCT/JP2005/012177
(87) International publication number: WO 2006/004028

(57) **Abstract**

The present invention relates to a pharmaceutical solution comprising the following components (A) and (B):
(A) a compound represented by the following formula (1): 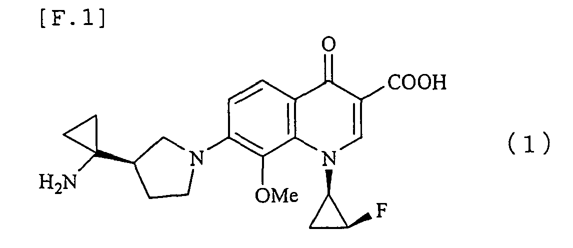 or a salt thereof, or a hydrate thereof; and
(B) a multivalent metal compound, wherein molar ratio of the component (B) to component (A) (the multivalent compound / component (B)) is 0.01 to 0.7. A pharmaceutical solution adapted for intravascular administration containing the quinolone compound at a therapeutically sufficient amount is provided, and this solution exhibits reduced toxicity even when the multivalent metal compound were incorporated at a reduced amount.

## Description

### [Field of the Invention]

This invention relates to a pharmaceutical solution containing a quinolone compound which exhibits reduced irritation to its injection site, particularly to the blood vessel, when it is administered to a patient, and also to a method for reducing irritation of the pharmaceutical solution containing the quinolone compound to its injection site, particularly to the blood vessel.

### [Background of the Invention]

Quinolone compounds exhibits excellent pharmaceutical effects as an antibacterial agent. However, when an aqueous injection solution or like that, containing the quinolone compound is administered by intravascular administration, some quinolone compounds develop irritation as a side effect to the blood vessel at the injection site of the compound. There is a method known for reducing such irritation by incorporating in the aqueous injection solution a multivalent metal compound which is a compound of the metal, such as magnesium, to promote formation of a chelate between the quinolone compound and the metal (ion).

More specifically, a pharmaceutical composition adaptable for intravenous administration containing the complex of quinolone carboxylic acid - metal ion - acid has been reported (see Patent document 1). A pharmaceutical solution capable of improving tolerance of an injected site has also been reported, and this solution contains a mixture of a quinolone compound or its pharmaceutically acceptable salt and a magnesium compound or a zinc compound with a cosolvent (see Patent document 2). Also reported is a preparation for subcutaneous or intramuscular administration containing a quinolone compound and magnesium ion, and this preparation exhibits reduced local irritation (see Patent document 3).

These preparations, however, required incorporation of the multivalent metal compound at a large amount in relation to the content of the quinolone compound. In the case of the Patent document 1, for example, the multivalent metal compound is incorporated at a molar ratio 1.5 to 3 times higher than the quinolone compound, and in the case of the Patent document 3, its advantageous effects tends to weaken unless the multivalent metal compound is used at least at an equimolar amount.

The metal (ion), however, has various physiological effects by itself, and limitation in the amount of the metal (ion) is mostly needed to avoid developments of such physiological effects. In the case of the previous pharmaceutical solutions having a large amount of the metal (ion) incorporated therein, there is an inevitable drawback in that the limitation of the total administration amount of the metal (ion) could lead to the limitation of the amount of the quinolone compound itself. If the amount of quinolone compound administered is limited in the serious infection case requiring the administration of a large amount of quinolone compound, there may be such a risk that only insufficient therapeutic effect is achieved by the administration of the quinolone compound.

In the meanwhile, a formulation has been reported whose metal (ion) content can be somewhat reduced (see Patent document 2). The characteristic feature of this formulation lies in the inclusion of a cosolvent such as N-methylpyrrolidone or 2-pyrrolidone. This formulation, however, is designed for subcutaneous injection, and the cosolvents mentioned in this document are not those normally used in the pharmaceutical solution for intravascular administration.

Furthermore, most solutions used for administering the quinolone compound are substantially neutral in pH to facilitate the chelate formation between the quinolone compound and the metal ion (Patent documents 1 to 3). A solution used for the administration, however, preferably has a weakly acidic pH of about 4 in view of the chemical and physicochemical stability of the quinolone compound. In other words, previous preparations of the quinolone compound have been well suited for chelate formation and showed good chelate formation ability while it is approximately neutral not necessarily ideal for the stability of the quinolone compound.

The quinolone compound (7-[3-(R)-(1-aminocyclopropyl)-pyrrolidine-1-yl]-1-[2-(S)-fluoro-1-(R)-cyclopropyl]-1,4-dihydro-8-methoxy-4-oxoquinoline-3-carboxylic acid) having the structure represented by the following formula (1):

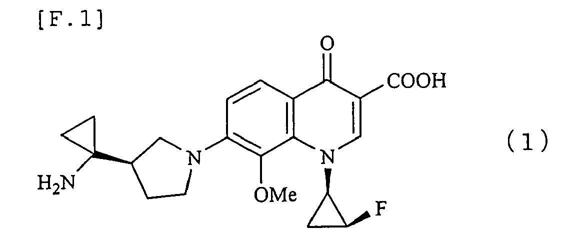

exhibits excellent antibacterial activities particularly for drug-resistant Gram-positive bacteria such as methicillin-resistant *Staphylococcus aureus,* penicillin-resistant-Streptococcus *pneumonie,* and vancomycin-resistant enterococci. This compound also shows higher safety, and is expected to show excellent therapeutic effects (see Patent document 4). Because of its antibacterial profile, this quinolone compound is also expected to exhibit excellent therapeutic effects in the case of serious infections in which the compound is generally administered in the form of a solution by intravascular administration. This in turn means that a formulation for a pharmaceutical solution containing this quinolone compound is required that enables intravascular administration with little or significantly reduced irritation to the injection site of administration when such pharmaceutical solution is administered by intravascular administration.
[Patent document 1] JP-A-05-502879
[Patent document 2] JP-A-11-501331
[Patent document 3] WO99/29322
[Patent document 4] WO02/40478

### [Disclosure of the Invention]

### [Problems to be solve by the Invention]

An object of the present invention is to provide a pharmaceutical solution adaptable for intravascular administration containing the quinolone compound at an amount therapeutically sufficient for treating an infectious disease, and which exhibits reduced local toxicity even when the multivalent metal compound is incorporated at a reduced amount.

The inventors of the present invention have made an extensive study to solve the problems as described above, and found that, if the multivalent metal compound is incorporated at a small amount in a pharmaceutical solution containing the particular predetermined quinolone compound, local toxicity, and in particular, irritation to the blood vessel can be alleviated even when the pharmaceutical solution is weakly acidic. The present invention has been accomplished on the bases of such finding.

The present invention provides a pharmaceutical solution comprising the following components (A) and (B):
(A) a compound represented by the following formula (1):

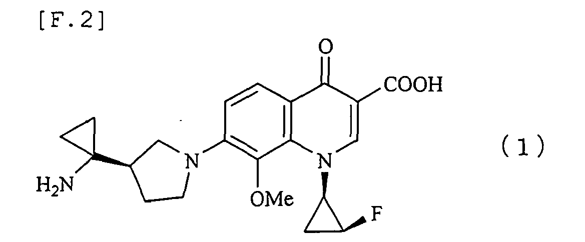

or a salt thereof; and
(B) a multivalent metal compound, wherein molar ratio of the component (B) to the component (A) (the multivalent compound / component (A)) is 0.01 to 0.7.
   This invention also provides;
   a preparation for preparing the pharmaceutical solution of the present invention containing the component (A) and the multivalent metal compound;
   a set for preparing the pharmaceutical solution of the present invention comprising a combination of a preparation containing the component (A), and a solution preparation containing the multivalent metal compound;
   a set for preparing the pharmaceutical solution of the present invention comprising a combination of a preparation containing the component (A) and the multivalent metal compound, and a reconstitution solution; and
   a set for preparing the pharmaceutical solution of the present invention comprising a combination of a solution preparation containing the component (A) and a solution preparation containing the multivalent metal compound.
   The present invention also provides a method for reducing irritation induced at the site of injection at the administration of a pharmaceutical solution containing component (A) which is a compound represented by the formula (1) or a salt thereof, or a hydrate thereof, in which the reduction is accomplished by incorporating a multivalent metal compound in the pharmaceutical solution containing the component (A), and the multivalent metal compound is incorporated at a molar ratio to the compound (A) (the multivalent metal / the component (A)) of 0.01 to 0.7.

### [Effects of the Invention]

The pharmaceutical solution of the present invention is capable of reducing the vascular toxicity of the quinolone compound represented by the formula (1) even when the pharmaceutical solution is weakly acidic. In the pharmaceutical solution of the present invention, the quinolone compound at a content sufficient to expect the high therapeutic effect can be incorporated in the pharmaceutical solution by incorporating the multivalent metal compound at a molar content as low as 0.01 to 0.7 times that of the quinolone compound.

### [Best Modes for carrying out the Invention]

The pharmaceutical solution of the present invention contains particular amounts of the component (A): the compound represented by the formula (1), its salt, or its hydrate (hereinafter referred to as "Compound (1)") and component (B): multivalent metal compound. Of the Compound (1), the compound (1) in the form of a hydrate is found only in the solid preparation, and this form is not in the pharmaceutical solution.

Compound (1) and the multivalent metal compound are not always required to coexist in the pharmaceutical solution at the time of the production of such pharmaceutical solution as long as they are simultaneously present in the pharmaceutical solution at the time of the administration. The pharmaceutical solution of the present invention may be in the form of, for example;
(i) a solution preparation in which both the Compound (1) and the multivalent metal compound are simultaneously dissolved;
(ii) a set comprising a combination of a solution preparation containing the Compound (1) dissolved therein and a solution preparation containing the multivalent metal compound dissolved therein; or
(iii) a set comprising a solid preparation containing the Compound (1) combined with a reconstitution solution to be used immediately before the administration for reconstituting the solid preparation.
   In the case of the preparation including the reconstitution solution, the multivalent metal compound may be incorporated in the reconstitution solution; in the preparation containing the Compound (1); in the reconstitution solution or in the preparation containing the Compound (1), and further in the diluent used for diluting the solution reconstituted by using the reconstitution solution; or only in the diluent and not in the reconstitution solution. The preparation containing the Compound (1) is not particularly limited as long as it can be used by the reconstitution immediately before the administration. The preparation containing the Compound (1), however, is preferably a lyophilized product.

More specifically, embodiments of the pharmaceutical solution of the present invention include those described below.

### Embodiment 1: Solution preparation

(I) A solution preparation containing both the Compound (1) and the multivalent metal compound dissolved in the solution, or (II) a set comprising a solution preparation containing only the Compound (1) dissolved therein without multivalent metal compound, and a solution preparation containing the multivalent metal compound (respectively corresponding to the above (i) and (ii)).

A pH adjusting agent, described later, may be further added to these solution preparations to adjust the pH of the solution used for administration to a weakly acidic pH of approximately 4.

In the case of (I), the preparation is produced as a solution simultaneously containing the Compound (1) and the multivalent metal ion derived from the multivalent metal compound.
In the case of (II), the multivalent metal compound is not added to the preparation containing the Compound (1), and this preparation is combined with a solution preparation containing the multivalent metal compound. This solution preparation containing the multivalent metal ion may be, for example, a commercially available infusion solution containing the multivalent metal ion used in the present invention. Accordingly, a solution preparation containing the Compound (1) but not the multivalent metal ion, aimed at being used as such a commercially available infusion solution containing the multivalent metal ion, is also within the embodiment of the present invention. In the case of (II), the set may further comprise a diluent which may contain the multivalent metal ion used in the present invention. The diluent containing the multivalent metal ion may be, for example, a commercially available infusion solution.

### Embodiment 2: Lyophilized product + reconstitution solution and/or diluent (the multivalent compound is incorporated either in one or both of these constituents)

A preparation containing the Compound (1), which is preferably a lyophilized product (optionally further containing a pH adjusting agent) combined with a solution containing the multivalent metal ion, the solution containing the multivalent metal ion being included as a reconstitution solution for the lyophilized product and/or a diluent (corresponding to the above (iii))
The reconstitution solution and/or the diluent may optionally have a pH adjusting agent added thereto to thereby render the solution used for the administration neutral.

In this case, the multivalent metal compound is not added to the preparation containing the Compound (1), but in the reconstitution solution for the lyophilized product and/or the diluent. The diluent containing the multivalent metal ion may be the diluent prepared specially for this product. The diluent, however, may be a commercially available infusion solution containing the multivalent metal ion used in the present invention. Accordingly, also included within the embodiment of the present invention is a set comprising the lyophilized product containing the Compound (1) combined with a reconstitution solution or a diluent which also serve the reconstitution solution, wherein the set is to be used with such commercially available infusion solution containing the multivalent metal ion.

### Embodiment 3: Lyophilized product containing multivalent metal ion + reconstitution solution and/or diluent

A preparation comprising a combination of a preparation containing the Compound (1) and the multivalent metal ion, which is preferably a lyophilized product, and a reconstitution solution and/or a diluent (corresponding to the above (iii)).
A pH adjusting agent(s) may be further added to such preparation to adjust the pH of the solution used for administration to a weakly acidic pH of approximately 4.

In this case, the lyophilized product contains the Compound (1) and the multivalent metal ion. The pharmaceutical solution of the present invention can be prepared by adding the reconstitution solution and/or the diluent to the lyophilized product containing the multivalent metal ion. The diluent used may be the one prepared specially for this product or a commercially available infusion solution or physiological saline, and the like.

At the administration, the solution preparation or the solution prepared by using the reconstitution solution may be further diluted by an infusion solution commonly used in the art. In this case, the solution may be diluted beforehand to thereby administer the diluted solution, or alternatively, the solution may be diluted by using a device like three way stopcock immediately before the Compound (1) starts its entering into the body.

The multivalent metal compound used in the present invention is not particularly limited as long as it interacts with the Compound (1) by its coexistence with the Compound (1) in the pharmaceutical solution. As an example of such interaction, formation of a metal chelate from the Compound (1) and the multivalent metal ion can be mentioned. In this case, the metal chelate may further comprise an anionic component of the pharmaceutical solution as one of its constituents.

In the present invention, both the quinolone compound and the multivalent metal ion may be dissolved in the solution, then interacting with each other before being administered. In the technique of the present invention, however, the amount of the multivalent metal required to cause the effect of reducing the irritation is smaller than the amount of multivalent metal normally conceived necessary for all of the quinolone compound incorporated to proceed the reaction of the metal chelate formation. Accordingly, it is conceived that the effect of the toxicity reduction can be attained even if only a part of the quinolone compound has formed the metal chelate. In other words, the interaction that may take place is not limited to the metal chelate formation, and the interaction may also be, for example, an interaction wherein a complex other than metal chelate is formed.

Such multivalent metal compound is preferably a magnesium compound or a calcium compound, and in particular, a magnesium compound. The magnesium compound and the calcium compound may be used in the form of a salt of such metal with an acid, or in the form of a halide or an oxide of such metal.

Examples of such salts with an acid include a salt with an organic acid (for example, a carboxylic acid such as gluconic acid or saccharic acid; or a sulfonic acid), and a salt with an inorganic acid (for example, sulfuric acid, hydrochloric acid, or hydrobromic acid). Examples of the salts with an organic acid include magnesium gluconate, calcium gluconate, and calcium saccharate, and exemplary salts with an inorganic acid include magnesium sulfate and magnesium calcium. Examples of the halide or oxide of the metal include magnesium chloride, calcium chloride, calcium bromide, and calcium oxide.

Of the multivalent metal compounds, the preferred one is a halide of magnesium or calcium, the more preferred one is a chloride of magnesium or calcium, and the most preferred one is magnesium chloride.

The multivalent metal compound may be incorporated at a molar ratio to the Compound (1) (multivalent metal / component (A)) of 0.01 to 0.7, more preferably at 0.016 to 0.7, still more preferably at 0.2 to 0.7, and most preferably at 0.26 to 0.6 in view of reducing the local irritation and incorporating the Compound (1) at an amount sufficient for realizing higher therapeutic effects.

The pharmaceutical solution of the present invention may have a pH range of 2.5 to 6.9, more preferably 3.5 to 6.5, still more preferably 3.0 to 5.5, and most preferably 3.5 to 4.5.

If desired, the preparation of the present invention may include additives which are typically included in a preparation for intravascular administration, and in particular, a preparation for intravenous administration. Exemplary, such additives include pH adjusting agent, isotonic agent, solubilizer, and pain reliever. When the pharmaceutical solution of the present invention has a reconstitution solution attached thereto, such additives may be added either to the preparation containing the Compound (1) or to the reconstitution solution.

Examples of the pH adjusting agents include inorganic acids such as hydrochloric acid, sulfuric acid, and phosphoric acid; inorganic acid salts such as sodium hydrogen carbonate, sodium carbonate, disodium hydrogen phosphate, sodium dihydrogen phosphate, trisodium phosphate, dipotassium hydrogen phosphate, potassium dihydrogen phosphate, sodium sulfite, sodium hydrogen sulfite, and sodium thiosulfate; organic acids such as acetic acid, lactic acid, succinic acid, maleic acid, tartaric acid, citric acid, ascorbic acid, salicylic acid, benzoic acid, methanesulfonic acid, and thioglycolic acid; organic acid ester compounds such as ethyl lactate; organic acid salts such as sodium citrate, disodium citrate, sodium gluconate, calcium citrate, sodium lactate, sodium acetate, sodium pyrophosphate, sodium benzoate, sodium caprylate, and sodium thioglycolate; inorganic acid salts such as sodium hydroxide; and organic amine compounds such as monoethanolamine, diethanolamine, triethanolamine, ethylenediamine, meglumine, and trometamol.

Examples of the isotonic agents include sugars and sugar alcohols such as sorbitol, mannitol, inositol, xylitol, erythritol, glucose, sucrose, refined white sugar, dextran, lactose, and maltose; and inorganic salts such as sodium chloride, and sodium bromide, or the like.

Examples of the solubilizers include sodium deoxycholate, polyoxyethylene hydrogenated castor oil, polyoxyethylene polyoxypropylene glycol, and Polysorbate 80, or the like.

Examples of the pain relievers include lidocaine and lidocaine hydrochloride, or the like.

The pharmaceutical solution of the present invention may be produced by following method commonly used in the art. More specifically, any method commonly used in the art may be employed as long as both the Compound (1) and the multivalent metal ion are simultaneously dissolved in the pharmaceutical solution. For example, it may be produced as a solution preparation containing the Compound (1) and the multivalent metal ion; or as a combination of a plurality of solution preparations respectively containing the Compound (1) and the multivalent metal ion; or alternatively, as a kit in which a preparation containing the Compound (1) is produced by lyophilization or the like, and this product is reconstituted with a reconstitution solution and/or a diluent immediately before the administration. In the latter case, the multivalent metal compound may be added either in the reconstitution solution and/or the diluent, or in the preparation containing the Compound (1).

In the production of the lyophilized product, the production is usually accomplished by adding an isotonic agent as mentioned above. However, in the lyophilized product containing Compound (1) of the Embodiment 2, addition of only the pH adjusting agent and not the isotonic agent to the Compound (1) is preferable in view of the stability. In addition, concentration of the Compound (1) in the solution used for producing the lyophilized product is preferably adjusted to the range of at least 10 mg/mL, more preferably to at least 15 mg/mL, and most preferably to at least 20 mg/mL in view of the cake formation. Also, the solution used for producing the lyophilized product should preferably have a pH of up to 5.5, more preferably up to 5, and most preferably 2.5 to 4.5 in view of the solubility of the Compound (1).

Next, exemplary productions are described.

### Embodiment 1: Solution

The Compound (1) and the multivalent metal compound are added and dissolved in the solvent commonly used in this field. When desired, pH adjusting agent may be added for pH adjustment, and when desired, other additives may be added and dissolved to produce the preparation. Immediately before the administration, this preparation is diluted to an adequate degree to prepare the solution for administration.
In alternative method, the Compound (1) and the pH adjusting agent are added and dissolved in the solvent. When desired, pH may be further adjusted, and other additives may be added and dissolved in the solution to produce the solution. In the meanwhile, the multivalent metal compound is added and dissolved in a solvent optionally with a pH adjusting agent and other additives to produce the preparation. Immediately before the administration, these preparations are mixed, and diluted to an adequate degree to thereby prepare the solution for administration.

Embodiment 2: Lyophilized product + reconstitution solution containing multivalent metal ion and/or diluent containing multivalent metal ion (the multivalent compound is incorporated in one or both of these constituents)
The Compound (1) and the pH adjusting agent are added and dissolved in the solvent. When desired, pH may be further adjusted, and when desired, other additives may be added and dissolved. The solution is then lyophilized to produce the lyophilized product. Immediately before the administration, the lyophilized product is reconstituted with a solution containing the multivalent metal compound, and diluted to an adequate degree to thereby prepare the solution for administration.
Alternatively, the lyophilized product may be reconstituted immediately before the administration with the reconstitution solution, and further diluted with a diluent containing the multivalent metal ion to thereby prepare the solution for administration.
Alternatively, a solution for administration having a neutral pH can be produced if a solution containing a pH adjusting agent is used for the reconstitution and/or the dissolution.

### Embodiment 3: Lyophilized product containing multivalent metal ion + reconstitution solution

The Compound (1) and the multivalent metal compound are added in a solvent for dissolution. When desired, pH may be adjusted, and when desired, other additives may be added to the solution for dissolution. The solution is then lyophilized to prepare the lyophilized product. Immediately before use, this product is reconstituted to an adequate degree to thereby prepare the solution for administration.
Alternatively, a solution for administration having an acidic pH can be produced if a solution containing a pH adjusting agent is used for the reconstitution and/or the dissolution.

The pharmaceutical solution of the present invention has enabled to reduce toxicity to the administration site, and in particular, toxicity to the blood vessel of the pharmaceutical solution.

When the pharmaceutical solution of the present invention is used as a drug to be administered to the human, the dose may vary according to the age, weight, type of the infection disease, seriousness of the infection, and the like. The dose (calculated in terms of the compound (1)), however, is typically 50 mg to 1 g, and preferably 100 mg to 800 mg per day per adult. When it is administered to an animal, the daily dose (calculated in terms of the compound (1)) is typically 1 to 200 mg, and preferably 5 to 1000 mg per kg of the body weight of the animal although the dose may vary depending on the size and the like of the animal to be treated. This daily dose may be administered in a single dose or 2 to 4 divided doses.

### [Examples]

Next, the present invention is described in further detail by referring to the Examples which by no means limit the scope of the present invention.
In the following Examples, the Compound (1) used was 7-[3-(R)-(1-aminocyclopropyl)pyrrolidine-1-yl]-1-[2-(S)-fluoro-1-(R)-cyclopropyl]-1,4-dihydro-8-methoxy-4-oxoquinoline-3-carboxylic acid.

### [Example 1] Lyophilized product

To water for injection (25 L) was added 1 mol/L hydrochloric acid (1400 mL), and the Compound (1) (600 g) was dissolved in this aqueous solution. To this solution was added 1 mol/L of hydrochloric acid to adjust the pH to 3.4, and then, water for injection to adjust the content of the Compound (1) to 20 mg/mL. The solution was filled in containers at 10 mL per container, and after lyophilization, the container was tightly sealed.

### [Example 2] Reconstitution solution (magnesium chloride solution)

Magnesium chloride (2.67 g) was dissolved by adding water for injection, and water for injection was further added to a total volume of 500 mL.

### [Example 3] Diluted reconstituted solution of the lyophilized product

The lyophilized product prepared in Example 1 was reconstituted with the magnesium chloride solution (10 mL) prepared in Example 2, and this solution was added to physiological saline (200 mL).

### [Example 4] Reconstitution solution (magnesium chloride-mannitol solution)

Magnesium chloride (5.34 g) and mannitol (10 g) was dissolved by adding water for injection, and water for injection was further added to a total volume of 1000 mL.

### [Example 5] Diluted reconstituted solution of the lyophilized product

The lyophilized product prepared in Example 1 was reconstituted with the magnesium chloride-mannitol solution (10 mL) prepared in Example 4, and this solution was added to physiological saline (200 mL).

### [Example 6] Reconstitution solution (magnesium chloride solution)

Magnesium chloride (3738 mg) was dissolved in water for injection (630 mL), and the solution was adjusted to 5.34 mg/mL with water for injection. This aqueous solution was filtered through a membrane filter, and the filtrate was filled in containers at 10 mL per container. After tight sealing, final sterilization was conducted at 121°C for 20 minutes.

### [Example 7] Reconstitution solution (magnesium chloride solution)

To water for injection (630 mL) was added 1 mol/L hydrochloric acid (2.2 mL). After dissolving magnesium chloride (3738 mg) in this solution, the solution was adjusted to pH 3.5 by adding 0.1 mol/L hydrochloric acid or 0.1 mol/L sodium hydroxide, and to 5.34 mg/mL with water for injection. The solution was filtered through a membrane filter, and filled in containers at 10 mL per container. After tight sealing, final sterilization was conducted at 121°C for 20 minutes.

### [Example 8] Reconstitution solution (magnesium chloride-mannitol solution)

2.5 g of magnesium chloride and 14.5 g of mannitol were accurately measured, and were dissolved in 450 mL of water. To this solution was added hydrochloric acid to adjust the pH to 2.5, 3.5, or 4.5, and water was added accurately to a final volume of 500 mL. The magnesium chloride-mannitol solution was then filtered through a filter, and the filtrate was filled in containers at 10 mL per container. After tight sealing, sterilization was conducted at 121°C for 20 minutes.

### [Example 9] Reconstitution solution (magnesium chloride-mannitol solution)

1.5 g of magnesium chloride and 16.5 g of mannitol were accurately measured, and were dissolved in 450 mL of water. To this solution was added hydrochloric acid to adjust the pH to 2.5, 3.5, or 4.5, and water was added accurately to a final volume of 500 mL. The magnesium chloride-mannitol solution was then filtered through a filter, and the filtrate was filled in containers at 10 mL per container. After tight sealing, sterilization was conducted at 121°C for 20 minutes.

### [Example 10] Reconstitution solution (magnesium chloride-mannitol solution)

650 mg of magnesium chloride and 18.5 g of mannitol were accurately measured, and were dissolved in 450 mL of water. To this solution was added hydrochloric acid to adjust the pH to 2.5, 3.5, or 4.5, and water was added accurately to a final volume of 500 mL. The magnesium chloride-mannitol solution was then filtered through a filter, and the filtrate was filled in containers at 10 mL per container. After tight sealing, sterilization was conducted at 121°C for 20 minutes.

### [Example 11] Reconstitution solution (magnesium chloride-sorbitol solution)

2.5 g of magnesium chloride and 14.5 g of sorbitol were accurately measured, and were dissolved in 450 mL of water. To this solution was added hydrochloric acid to adjust the pH to 2.5, 3.5, or 4.5, and water was added accurately to a final volume of 500 mL. The magnesium chloride-sorbitol solution was then filtered through a filter, and the filtrate was filled in containers at 10 mL per container. After tight sealing, sterilization was conducted at 121°C for 20 minutes.

### [Example 12] Reconstitution solution (magnesium chloride-sorbitol solution)

1.5 g of magnesium chloride and 16.5 g of sorbitol were accurately measured, and were dissolved in 450 mL of water. To this solution was added hydrochloric acid to adjust the pH to 2.5, 3.5, or 4.5, and water was added accurately to a final volume of 500 mL. The magnesium chloride-sorbitol solution was then filtered through a filter, and the filtrate was filled in containers at 10 mL per container. After tight sealing, sterilization was conducted at 121°C for 20 minutes.

### [Example 13] Reconstitution solution (magnesium chloride-sorbitol solution)

650 mg of magnesium chloride and 18.5 g of sorbitol were accurately measured, and were dissolved in 450 mL of water. To this solution was added hydrochloric acid to adjust the pH to 2.5, 3.5, or 4.5, and water was added accurately to a final volume of 500 mL. The magnesium chloride-sorbitol solution was then filtered through a filter, and the filtrate was filled in containers at 10 mL per container. After tight sealing, sterilization was conducted at 121°C for 20 minutes.

### [Example 14] Diluted reconstituted solution of the lyophilized product (magnesium chloride-mannitol solution)

2.5 g of magnesium chloride and 14.5 g of mannitol were accurately measured, and they dissolved in 450 mL of water. To this solution was added hydrochloric acid to adjust the pH to 3.5, and water was added accurately to a final volume of 500 mL. The solution was filtered, and the lyophilized product prepared in Example 1 was reconstituted with 10 mL of the filtered magnesium chloride-mannitol solution. After reconstituting two containers of lyophilized product prepared in Example 1, 20 mL of the reconstituted solution was diluted with physiological saline to a final volume of 100 mL.

### [Example 15] Diluted reconstituted solution of the lyophilized product (magnesium chloride-sorbitol solution)

2.5 g of magnesium chloride and 14.5 g of sorbitol were accurately measured, and were dissolved in 450 mL of water. To this solution was added hydrochloric acid to adjust the pH to 3.5, and water was added accurately to a final volume of 500 mL. The solution was filtered, and the lyophilized product prepared in Example 1 was reconstituted with 10 mL of the filtered magnesium chloride-sorbitol solution. After reconstituting two vials of lyophilized product prepared in Example 1, 20 mL of the reconstituted solution was diluted with physiological saline to a final volume of 100 mL.

### [Example 16] Reconstitution solution (5% sorbitol solution containing magnesium chloride)

1.5 g magnesium chloride and 75 g of sorbitol were accurately measured, and after adding water accurately to a final volume of 1500 mL, the solution was filtered through a filter. The 5% sorbitol solution containing the magnesium chloride was filled in containers at 100 mL per container. After tight sealing, sterilization was conducted at 122°C for 30 minutes.

### [Example 17] Diluted reconstituted solution of the lyophilized product (5% sorbitol solution containing magnesium chloride)

20 mL of the 5% sorbitol solution containing magnesium chloride produced in Example 16 was aliquoted. Two containers of the lyophilized product prepared in Example 1 were respectively reconstituted with 10 mL of the 5% sorbitol solution containing magnesium chloride. The resulting 20 mL reconstituted solution was returned to the product of Example 16.

### [Example 18] Diluted reconstituted solution of the lyophilized product (magnesium chloride-mannitol solution)

0.3 g of magnesium chloride and 3.3 g of mannitol were accurately measured, and were dissolved in 90 mL of water. To this solution was added hydrochloric acid to adjust the pH to 3.5, and water was added accurately to a final volume of 100 mL. The solution was filtered, and the lyophilized product prepared in Example 1 was reconstituted with 10 mL of the filtered magnesium chloride-mannitol solution. 10 mL of the reconstituted solution was diluted with physiological saline to a final volume of 50 mL.

### [Example 19] Diluted reconstituted solution of the lyophilized product (magnesium chloride-sorbitol solution)

0.3 g of magnesium chloride and 3.3 g of sorbitol were accurately measured, and were dissolved in 90 mL of water. To this solution was added hydrochloric acid to adjust the pH to 3.5, and water was added accurately to a final volume of 100 mL. The solution was filtered, and the lyophilized product prepared in Example 1 was reconstituted with 10 mL of the filtered magnesium chloride-sorbitol solution. 10 mL of the reconstituted solution was diluted with physiological saline to a final volume of 50 mL.

### [Example 20] Reconstitution solution (5% sorbitol solution containing magnesium chloride)

0.18 g magnesium chloride and 15 g of sorbitol were accurately measured, and after adding water accurately to a final volume of 300 mL, the solution was filtered through a filter. The 5% sorbitol solution containing the magnesium chloride was filled in containers at 50 mL per container. After tight sealing, sterilization was conducted at 122°C for 30 minutes.

### [Example 21] Diluted reconstituted solution of the lyophilized product (5% sorbitol solution containing magnesium chloride)

10 mL of the 5% sorbitol solution containing magnesium chloride produced in Example 20 was aliquoted, and used to reconstitute the lyophilized product prepared in Example 1. The reconstituted solution was returned to the product of Example 20.

### [Preparation Example 1]

D-sorbitol (6 g) and magnesium chloride (1.05 g) were dissolved in water for injection (100 mL). After adding 0.5 mol/L hydrochloric acid (0.75 mL), the Compound (1) (3.0 g) was dissolved to this solution, and pH was adjusted to 6.0 by adding 0.5 mol/L hydrochloric acid. Water for injection was then added to a final volume of 150 mL.

### [Preparation Example 2]

40 parts of the solution prepared in Preparation Example 1 and 60 parts of physiological saline were mixed to prepare solution for administration (Concentration of the Compound (1): 8 mg/mL).

### [Preparation Example 3]

To the Compound (1) (200 mg) were added 10 w/v% aqueous solution of mannitol (3.5 mL) and 2.5 w/v% aqueous solution of magnesium chloride (2.8 mL) to dissolve the Compound (1). Water for injection (3.7 mL) was then added.

### [Preparation Example 4]

The solution of Preparation Example 3 (7 mL) was mixed with physiological saline (10.5 mL).

### [Preparation Example 5]

The solution of Preparation Example 4 (6 mL) was mixed with physiological saline (6 mL).

### [Preparation Example 6]

To the Compound (1) (200 mg) were added 10 w/v% aqueous solution of mannitol (4 mL) and 2.5 w/v% aqueous solution of magnesium chloride (2.12 mL) to dissolve the Compound (1). Water for injection (3.88 mL) was then added.

### [Preparation Example 7]

The solution of Preparation Example 6 (7 mL) was mixed with physiological saline (10.5 mL).

### [Preparation Example 8]

The solution of Preparation Example 7 (6 mL) was mixed with physiological saline (6 mL).

### [Preparation Example 9]

Lyophilized product prepared in Example 1 was reconstituted with water for injection (5 mL). To this solution were added 10 w/v% aqueous solution of mannitol (2 mL), 2.5 w/v% aqueous solution of magnesium chloride (2.8 mL), and water for injection (0.2 mL). This solution (7 mL) was mixed with physiological saline (10.5 mL).

### [Preparation Example 10]

The solution of Preparation Example 9 (6 mL) was mixed with physiological saline (6 mL).

### [Preparation Example 11]

Lyophilized product prepared in Example 1 was reconstituted with water for injection (5 mL). To this solution were added 10 w/v% aqueous solution of mannitol (2.5 mL), 2.5 w/v% aqueous solution of magnesium chloride (2.12 mL), and water for injection (0.38 mL)

### [Preparation Example 12]

The solution of Preparation Example 11 (7 mL) was mixed with physiological saline (10.5 mL).

### [Preparation Example 13]

The solution of Preparation Example 12 (6 mL) was mixed with physiological saline (6 mL).

### [Preparation Example 14]

To the Compound (1) (3.168 g) was added 0.06 mol/L hydrochloric acid to dissolve the Compound (1), and 0.06 mol/L hydrochloric acid was further added to adjust the pH to 3.5. Water for injection was added to this solution to a final volume of 151.8 mL. To this solution (23 mL) were added 12.72 w/v% aqueous solution of magnesium chloride (0.5 mL), water for injection (0.5 mL), physiological saline containing 10 w/v% mannitol (8.4 mL), and physiological saline (27.6 mL), and the mixture was stirred.

### [Preparation Example 15]

To the Compound (1) (3.168 g) was added 0.06 mol/L hydrochloric acid to dissolve the Compound (1), and 0.06 mol/L hydrochloric acid was further added to adjust the pH to 3.5. Water for injection was added to this solution to a final volume of 151.8 mL. To 11.5 mL of this solution (total amount used including the solution used for the concentration adjustment: 11.85 mL) were added 12.72 w/v% aqueous solution of magnesium chloride (0.25 mL), water for injection (0.25 mL), physiological saline containing 10 w/v% mannitol (4.2 mL), and physiological saline (43.8 mL), and after stirring, the mixture was adjusted for its concentration.

### [Preparation Example 16]

To the Compound (1) (4.174 g) was added 0.06 mol/L hydrochloric acid to dissolve the Compound (1), and 0.06 mol/L hydrochloric acid was further added to adjust the pH to 3.5. Water for injection was added to this solution to a final volume of 200 mL. To this solution (46 mL) were added 12.72 w/v% aqueous solution of magnesium chloride (1 mL), water for injection (1 mL), physiological saline containing 10 w/v% mannitol (16.8 mL), and physiological saline (55.2 mL), and the mixture was stirred.

### [Preparation Example 17]

Physiological saline (40 mL) was added to 40 mL of the solution of the Preparation Example 16 (total amount used including the solution used for the concentration adjustment: 40.46 mL). After stirring, the mixture was adjusted for its concentration.

### [Preparation Example 18]

To the Compound (1) (3.168 g) was added 0.06 mol/L hydrochloric acid to dissolve the compound (1), and 0.06 mol/L hydrochloric acid was further added to adjust the pH to 3.5. Water for injection was added to this solution to a final volume of 151.8 mL. To 23 mL of this solution (total amount used including the solution used for the concentration adjustment: 23.58 mL) were added 12.72 w/v% aqueous solution of magnesium chloride (1.0 mL), physiological saline containing 10 w/v% mannitol (7.2 mL), and physiological saline (28.8 mL), and after stirring, the mixture was adjusted for the concentration of the Compound (1).

### [Preparation Example 19]

To the Compound (1) (3.168 g) was added 0.06 mol/L hydrochloric acid to dissolve the compound (1), and 0.06 mol/L hydrochloric acid was further added to adjust the pH to 3.5. Water for injection was added to this solution to a final volume of 151.8 mL. To 11.5 mL of this solution (total amount used including the solution used for the concentration adjustment: 11.85 mL) were added 12.72 w/v% aqueous solution of magnesium chloride (0.5 mL), physiological saline containing 10 w/v% mannitol (3.6 mL), and physiological saline (44.4 mL), and after stirring, the mixture was adjusted for the concentration of the Compound (1).

### [Preparation Example 20]

To the Compound (1) (4.174 g) was added 0.06 mol/L hydrochloric acid to dissolve the compound (1), and 0.06 mol/L hydrochloric acid was further added to adjust the pH to 3.5. Water for injection was added to this solution to a final volume of 200 mL. To this solution (46 mL) were added 12.72 w/v% aqueous solution of magnesium chloride (2 mL), physiological saline containing 10 w/v% mannitol (14.4 mL), and physiological saline (57.6 mL), and the mixture was stirred.

### [Preparation Example 21]

Physiological saline (40 mL) was added to 40 mL of the solution of Preparation Example 20 (total amount used including the solution used for the concentration adjustment: 40.46 mL), and after stirring, concentration of the Compound (1) was adjusted.

### [Preparation Example 22]

To the Compound (1) (3.168 g) was added 0.06 mol/L hydrochloric acid to dissolve the compound (1), and 0.06 mol/L hydrochloric acid was further added to adjust the pH to 3.5. Water for injection was added to this solution to a final volume of 151.8 mL. To this solution (23 mL) were added 33.6 w/v% aqueous solution of magnesium chloride (1.0 mL), physiological saline containing 10 w/v% mannitol (2.4 mL), and physiological saline (33.6 mL), and the mixture was stirred.

### [Preparation Example 23]

To the Compound (1) (3.168 g) was added 0.06 mol/L hydrochloric acid to dissolve the compound (1), and 0.06 mol/L hydrochloric acid was further added to adjust the pH to 3.5. Water for injection was added to this solution to a final volume of 151.8 mL. To this solution 11.5 mL (11.85 mL) were added 33.6 w/v% aqueous solution of magnesium chloride (0.5 mL), physiological saline containing 10 w/v% mannitol (1.2 mL), and physiological saline (46.8 mL), and the mixture was stirred.

### [Preparation Example 24]

To the Compound (1) (4.174 g) was added 0.06 mol/L hydrochloric acid to dissolve the compound (1), and 0.06 mol/L hydrochloric acid was further added to adjust the pH to 3.5. Water for injection was added to this solution to a final volume of 200 mL. To this solution (34.5 mL) were added 33.6 w/v% aqueous solution of magnesium chloride (1.5 mL), physiological saline containing 10 w/v% mannitol (3.6 mL), and physiological saline (50.4 mL), and the mixture was stirred.

### [Preparation Example 25]

Physiological saline (40 mL) was added to the solution of Preparation Example 24 (40 mL), and the mixture was stirred.

### [Preparation Example 26]

To the Compound (1) (4.592 g) was added 0.06 mol/L hydrochloric acid to dissolve the compound (1), and 0.06 mol/L hydrochloric acid was further added to adjust the pH to 3.5. Water for injection was added to this solution to a final volume of 220 mL. To this solution (51.75 mL) were added 12.72 w/v% aqueous solution of magnesium chloride (0.5625 mL), water for injection (1.6875 mL), physiological saline containing 10 w/v% mannitol (19.98 mL), and physiological saline (98.82 mL), and the mixture was stirred.

### [Preparation Example 27]

Physiological saline (56 mL) was added to the solution of Preparation Example 26 (56 mL), and the mixture was stirred.

### [Preparation Example 28]

To the Compound (1) (4.383 g) was added 0.06 mol/L hydrochloric acid to dissolve the compound (1), and 0.06 mol/L hydrochloric acid was further added to adjust the pH to 3.5. Water for injection was added to this solution to a final volume of 210 mL. To this solution (48.3 mL) were added 13.25 w/v% aqueous solution of magnesium chloride (0.504 mL), water for injection (1.596 mL), physiological saline containing 10 w/v% mannitol (18.648 mL), and physiological saline (92.232 mL), and the mixture was stirred.

### [Preparation Example 29]

Physiological saline (53 mL) was added to the solution of Preparation Example 28 (53 mL), and the mixture was stirred.

### [Preparation Example 30]

To the Compound (1) (5.009 g) was added 0.06 mol/L hydrochloric acid to dissolve the compound (1), and 0.06 mol/L hydrochloric acid was further added to adjust the pH to 3.5. Water for injection was added to this solution to a final volume of 240 mL. To this solution (57.5 mL) were added 13.25 w/v% aqueous solution of magnesium chloride (0.6 mL), water for injection (1.9 mL), physiological saline containing 10 w/v% mannitol (22.2 mL), and physiological saline (109.8 mL), and the mixture was stirred.

### [Preparation Example 31]

Physiological saline (64 mL) was added to the solution of Preparation Example 30 (64.5 mL), and the mixture was stirred.

### [Preparation Example 32]

To the Compound (1) (4.592 g) was added 0.06 mol/L hydrochloric acid to dissolve the compound (1), and 0.06 mol/L hydrochloric acid was further added to adjust the pH to 3.5. Water for injection was added to this solution to a final volume of 220 mL. To this solution (51.75 mL) were added 12.72 w/v% aqueous solution of magnesium chloride (1.125 mL), water for injection (1.125 mL), physiological saline containing 10 w/v% mannitol (18.9 mL), and physiological saline (99.9 mL), and the mixture was stirred.

### [Preparation Example 33]

Physiological saline (56 mL) was added to the solution of Preparation Example 32 (56 mL), and the mixture was stirred.

### [Preparation Example 34]

To the Compound (1) (4.383 g) was added 0.06 mol/L hydrochloric acid to dissolve the compound (1), and 0.06 mol/L hydrochloric acid was further added to adjust the pH to 3.5. Water for injection was added to this solution to a final volume of 210 mL. To this solution (48.3 mL) were added 13.25 w/v% aqueous solution of magnesium chloride (1.008 mL), water for injection (1.092 mL), physiological saline containing 10 w/v% mannitol (17.64 mL), and physiological saline (93.24 mL), and the mixture was stirred.

### [Preparation Example 35]

Physiological saline (53 mL) was added to the solution of Preparation Example 34 (53 mL), and the mixture was stirred.

### [Preparation Example 36]

To the Compound (1) (5.009 g) was added 0.06 mol/L hydrochloric acid to dissolve the compound (1), and 0.06 mol/L hydrochloric acid was further added to adjust the pH to 3.5. Water for injection was added to this solution to a final volume of 240 mL. To this solution (57.5 mL) were added 13.25 w/v% aqueous solution of magnesium chloride (1.2 mL), water for injection (1.3 mL), physiological saline containing 10 w/v% mannitol (21 mL), and physiological saline (111 mL), and the mixture was stirred.

### [Preparation Example 37]

Physiological saline (64 mL) was added to the solution of Preparation Example 36 (64 mL), and the mixture was stirred.

### [Preparation Example 38]

To the Compound (1) (4.592 g) was added 0.06 mol/L hydrochloric acid to dissolve the compound (1), and 0.06 mol/L hydrochloric acid was further added to adjust the pH to 3.5. Water for injection was added to this solution to a final volume of 220 mL. To this solution (51.75 mL) were added 12.72 w/v% aqueous solution of magnesium chloride (2.25 mL), physiological saline containing 10 w/v% mannitol (16.2 mL), and physiological saline (102.6 mL), and the mixture was stirred.

### [Preparation Example 39]

Physiological saline (56 mL) was added to the solution of Preparation Example 38 (56 mL), and the mixture was stirred.

### [Preparation Example 40]

To the Compound (1) (4.383 g) was added 0.06 mol/L hydrochloric acid to dissolve the compound (1), and 0.06 mol/L hydrochloric acid was further added to adjust the pH to 3.5. Water for injection was added to this solution to a final volume of 210 mL. To 48.3 mL of this solution (total amount used including the solution used for the concentration adjustment: 49 mL) were added 13.25 w/v% aqueous solution of magnesium chloride (2.016 mL), water for injection (0.084 mL), physiological saline containing 10 w/v% mannitol (15.12 mL), and physiological saline (95.76 mL), and after stirring, the mixture was adjusted for the concentration of the Compound (1).

### [Preparation Example 41]

Physiological saline (53 mL) was added to the solution of Preparation Example 40 (53 mL), and the mixture was stirred.

### [Preparation Example 42]

To the Compound (1) (5.009 g) was added 0.06 mol/L hydrochloric acid to dissolve the compound (1), and 0.06 mol/L hydrochloric acid was further added to adjust the pH to 3.5. Water for injection was added to this solution to a final volume of 240 mL. To this solution (57.5 mL) were added 13.25 w/v% aqueous solution of magnesium chloride (2.4 mL), water for injection (0.1 mL), physiological saline containing 10 w/v% mannitol (18 mL), and physiological saline (114 mL), and the mixture was stirred.

### [Preparation Example 43]

Physiological saline (64 mL) was added to the solution of Preparation Example 42 (64 mL), and the mixture was stirred.

### [Contrast Example 1]

Sodium chloride (1.656 g) was dissolved in water for injection (150 mL). To this solution was added 0.5 mol/L hydrochloric acid (22.425 mL), and then, the Compound (1) (5.0 g). 0.5 mol/L sodium hydroxide aqueous solution was further added to adjust the pH to 4.5, and water for injection was added to a final volume of 250 mL. 40 part of this solution was mixed with 60 parts of physiological saline (concentration of the Compound (1): 8 mg/mL).

### [Contrast Example 2]

To the Compound (1) (4.592 g) was added 0.06 mol/L hydrochloric acid to dissolve the compound (1), and 0.06 mol/L hydrochloric acid was further added to adjust the pH to 3.5. Water for injection was added to this solution to a final volume of 220 mL. To this solution (51.75 mL) were added water for injection (2.25 mL), physiological saline containing 10 w/v% mannitol (21.6 mL), and physiological saline (97.2 mL), and the mixture stirred (pH 4.5) .

### [Contrast Example 3]

Physiological saline (56 mL) was added to the solution of Contrast Example 2 (56 mL), and the mixture was stirred (pH 4.7).

### [Contrast Example 4]

To the Compound (1) (4.383 g) was added 0.06 mol/L hydrochloric acid to dissolve the compound (1), and 0.06 mol/L hydrochloric acid was further added to adjust the pH to 3.5. Water for injection was added to this solution to a final volume of 210 mL. To this solution (48.3 mL) were added water for injection (2.1 mL), physiological saline containing 10 w/v% mannitol (20.16 mL), and physiological saline (90.72 mL), and the mixture stirred (pH 4.2) .

### [Contrast Example 5]

Physiological saline (53 mL) was added to the solution of Contrast Example 4 (53 mL), and the mixture was stirred (pH 4.5).

### [Contrast Example 6]

To the Compound (1) (5.009 g) was added 0.06 mol/L hydrochloric acid to dissolve the compound (1), and 0.06 mol/L hydrochloric acid was further added to adjust the pH to 3.5. Water for injection was added to this solution to a final volume of 240 mL. To this solution (57.5 mL) were added water for injection (2.5 mL), physiological saline containing 10 w/v% mannitol (24 mL), and physiological saline (108 mL), and the mixture stirred (pH 4.0).

### [Contrast Example 7]

Physiological saline (64 mL) was added to the solution of Contrast Example 6 (64 mL), and the mixture was stirred (pH 4.3).

### [Test Example 1] (Repeated intravenous administration test in rat)

The pharmaceutical solution of Preparation Example 2, the pharmaceutical solution of Contrast Example 1, and physiological saline were respectively administered to rats for 9 days by intravenous administration (dosing volume, 10 mL/kg/day; administration rate, 1 mL/min), and the animals were sacrificed on the next day of the last administration to evaluate the local irritation. The pharmaceutical solution was basically administered to the right tail vein, and the left tail vein was used when the right tail vein could not be used. The sum of the number of administration in which the right tail vein could not be used and the score of the histological test results (the larger score means the stronger toxicity) was used for the evaluation. The results are shown in Table 1.

### [Test Example 2] (Repeated intravenous administration test in rabbit)

### <Test method of the repeated intravenous administration in rabbit>

An injection needle was inserted in rabbit auricular vein, and the part of the vein where the injection needle is inserted was clamped (site A). After injecting the pharmaceutical solution (0.05 mL), the part of the vein 3 cm central to the site A was clamped. After injecting the remaining pharmaceutical solution (0.05 mL), the injection needle was removed, and the clamp was removed after 3 minutes.

The pharmaceutical solution of Preparation Example 2, the pharmaceutical solution of Contrast Example 1, and physiological saline were respectively administered to rabbits for 14 days by the rabbit repeated intravenous administration (1) as described above (dosing volume, 0.1 mL/rabbit/day), and the animals were sacrificed on the next day of the last administration to evaluate the local toxicity. The score of the histological test results (the larger score means the stronger irritation) was used for the evaluation. The results are shown in Table 1.

### [Test Example 3] (Single intramuscular administration test in rabbit)

The pharmaceutical solution of Preparation Example 2, the pharmaceutical solution of Contrast Example 1, and physiological saline were respectively administered to femoral lateral vastus of a rabbit in single dose (dosing volume, 1 mL/site), and the animals were sacrificed 2 days after the last administration for evaluation.
The sum of the ratio of serum CK level after the administration to the serum CK level before the administration and the score of the histological test results (the larger score means the severe irritation) was used for the evaluation. The results are shown in Table 1.

**Table 1**

| Results of the evaluation of local irritation I | | | |
|---|---|---|---|
| | Test Example 1 | Test Example 2 | Test Example 3 |
| Preparation Example 2 | 2.2 | 0 | 18.8 |
| Contrast Example 1 | 8.4 | 2.0 | 25.2 |
| Physiological saline | 0.4 | 0 | 13.8 |

As demonstrated in Table 1, the pharmaceutical solution of the present invention exhibited lower irritation than the pharmaceutical solution of the of Contrast Example 1 in all of Test Examples 1 to 3, and toxicity was substantially equivalent to that of the physiological saline.

### [Test Example 4] (Repeated 2-week intravenous administration test in rat)

The pharmaceutical solutions of Preparation Examples 27, 29, 31, 33, 35, 37, 39, 41, 43, 26, 28, 30, 32, 34, 36, 38, 40, and 42 and Contrast Examples 2, 4, 6, 3, 5, and 7 were respectively administered to rats repeatedly from their tail vein once a day and 14 times in total (dosing volume, 1 mL/kg/day; administration rate, 1 mL/min; 5 animals per group). The animals were sacrificed and the administration site was observed with visually and subjected to histological test (7 samples per animal were collected from the vein which is the administration site, and the tissue finding was scored by the criteria of 0: no change, 1: slight, 2: moderate, and 3: severe). The results are shown in Table 2. The score of the histological test shown is the average of the scores.

As demonstrated in Table 2, while erosion (2 cases in 5 cases), crust (1 case in 5 cases), and swelling (5 cases in 5 cases) were confirmed at the site of administration in the case of high concentration pharmaceutical solutions (concentration of the Compound (1): 6.25 mg/mL) (Contrast Examples 2, 4, and 6), in the pharmaceutical solutions of the present invention having magnesium chloride added thereto (Preparation Examples 26, 28, and 30), these symptoms occurred less frequently (erosion, 1 case in 5 cases).
In particular, when the amount of the magnesium chloride added was 0.828 mg/mL or higher, no such symptoms were observed.
The pharmaceutical solutions of the present invention also showed scores lower than those of Contrast Examples, in the terms of necrosis and degeneration of the vascular wall as well as all other tissue findings. In particular, neither vascular wall necrosis nor vascular wall degeneration was recognized when the pharmaceutical solution of the present invention was used at low concentration (concentration of the Compound (1), 3.125 mg/mL).

Accordingly, it has been confirmed that the pharmaceutical solution of the present invention containing the magnesium at a molar ratio 0.01 to 0.7 times that of the solution is capable of reducing the local irritation of the Compound (1) to blood vessel irrespective of the pH of the pharmaceutical solution.

**Table 2**

| The results of local irritation evaluation 2 (Test Example 4) | | | | | | |
|---|---|---|---|---|---|---|
| Concentration of Compound (1) (mg/mL) | Concent ration of MgCl₂ (mg/mL) | Molar ratio of MgCl₂/ Compound (1) | Dosing solution | Results of visual observation | Results of histological test Score-1^{*1} | Results of histological test Score-1^{*2} |
| 3.125 | 0 | 0 | Contrast Ex. (3) | No change | 0.20 | 2.46 |
| | | | (5) (7) | | | |
| 3.125 | 0.207 | 0.13 | Prep. Ex. (27) (29) (31) | No change | 0 | 1.51 |
| 3.125 | 0.414 | 0.26 | Prep. Ex. (33) (35) (37) | No change | 0 | 1.71 |
| 3.125 | 0.828 | 0.52 | Prep. Ex. (39) (41) (43) | No change | 0 | 1.66 |
| 6.25 | 0 | 0 | Contrast Ex. (2) (4) (6) | Erosion (2 in 5) crust (1 in 5) swelling (5 in 5) | 0.66 | 7.17 |
| 6.25 | 0.414 | 0.13 | Prep. Ex. (26) (28) (30) | Erosion (1 in 5) | 0 | 1.83 |
| 6.25 | 0.828 | 0.26 | Prep. Ex. (32) (34) (36) | No change | 0.03 | 1.97 |
| 6.25 | 1.66 | 0.52 | Prep. Ex. (38) (40) (42) | No change | 0 | 1.43 |

| | | | | | | |
|---|---|---|---|---|---|---|
| *1 Score-1: Average score of necrosis and degeneration of vascular wall *2 Score-2: Average score of all tissue findings | | | | | | |

### [Contrast Example 8]

To the Compound (1) was added 0.06 mol/L hydrochloric acid to dissolve the compound (1), and 0.06 mol/L hydrochloric acid was further added to adjust the pH to 3.5. Water for injection was added to this solution to adjust the concentration of the Compound (1) to 20 mg/mL. To this solution (1 mL) were added physiological saline containing 10 w/v% mannitol (0.4 mL) and physiological saline (8.6 mL), and the mixture was stirred.

### [Preparation Example 44]

To the Compound (1) was added 0.06 mol/L hydrochloric acid to dissolve the compound (1), and 0.06 mol/L hydrochloric acid was further added to adjust the pH to 3.5. Water for injection was added to this solution to adjust the concentration of the Compound (1) to 20 mg/mL. To this solution (1 mL) were added 13.25% aqueous solution of magnesium chloride (0.01 mL), physiological saline containing 10 w/v% mannitol (0.37 mL) and physiological saline (8.62 mL), and the mixture was stirred.

### [Preparation Example 45]

To the Compound (1) was added 0.06 mol/L hydrochloric acid to dissolve the compound (1), and 0.06 mol/L hydrochloric acid was further added to adjust the pH to 3.5. Water for injection was added to this solution to adjust the concentration of the Compound (1) to 20 mg/mL. To this solution (1 mL) were added 13.25% aqueous solution of magnesium chloride (0.02 mL), physiological saline containing 10 w/v% mannitol (0.35 mL) and physiological saline (8.63 mL), and the mixture was stirred.

### [Preparation Example 46]

To the Compound (1) was added 0.06 mol/L hydrochloric acid to dissolve the compound (1), and 0.06 mol/L hydrochloric acid was further added to adjust the pH to 3.5. Water for injection was added to this solution to adjust the concentration of the Compound (1) to 20 mg/mL. To this solution (1 mL) were added 13.25% aqueous solution of magnesium chloride (0.04 mL), physiological saline containing 10 w/v% mannitol (0.3 mL) and physiological saline (8.66 mL), and the mixture was stirred.

### [Test Example 5] (Single intramuscular administration test in rabbit)

The pharmaceutical solutions of Preparation Examples 44, 45, and 46, the pharmaceutical solution of Contrast Example 8, and physiological saline were respectively administered to femoral lateral vastus of a rabbit in single dose (dosing volume, 1 mL/site), and the animals were sacrificed on 2 days after the last administration for evaluation. The administration site was observed visually (by collecting lateral vastus and lesions around the muscle and scoring the tissue findings by the criteria of 0: no change, 1: very slight, 2: slight, 3: moderate, and 4: severe) and subjected to histological test (by collecting the muscle to prepare the specimen and scoring the tissue change). The ratio of serum CK level after the administration to the serum CK level before the administration was also calculated. The results are shown in Table 3. The score of the histological test shown is the average of the scores.
As demonstrated in Table 3, the pharmaceutical solutions of the present invention having 0.27 mg/ml or more (0.26 or more in molar ratio to the Compound (1)) of magnesium chloride added thereto exhibited lower score than the Contrast Example in the visual observation and histological test.

**Table 3**

| Results of the evaluation of local toxicity (Test Example 5) | | | | | |
|---|---|---|---|---|---|
| Concent-ration of the Compound (1) (mg/mL) | MgCl₂ concentration (mg/mL) | Molar ratio of MgCl₂/ Compound (1) | Dosing solution | Results of visual observation, Score-1 | Results of histological test, Score-2 |
| 0 | 0 | - | Physiological saline | 0.5 | 3.5 |
| 2 | 0 | 0 | Contrast Example 8 | 3.0 | 6.8 |
| 2 | 0.13 | 0.13 | preparation Example 44 | 3.3 | 6.8 |
| 2 | 0.27 | 0.26 | Preparation Example 45 | 1.8 | 6.0 |
| 2 | 0.53 | 0.52 | Preparation Example 46 | 0.5 | 3.0 |

### [Contrast Example 9]

To the Compound (1) was added 0.06 mol/L hydrochloric acid to dissolve the Compound (1), and 0.06 mol/L hydrochloric acid was further added to adjust the pH to 3.5. Water for injection was added to this solution to adjust the concentration of the Compound (1) to 20 mg/mL. To this solution (43.75 mL) was added physiological saline containing 10 w/v% mannitol (17.5 mL) and physiological saline (78.75 mL), and the mixture was stirred.

### [Preparation Example 47]

To the Compound (1) was added 0.06 mol/L hydrochloric acid to dissolve the compound (1), and 0.06 mol/L hydrochloric acid was further added to adjust the pH to 3.5. Water for injection was added to this solution to adjust the concentration of the Compound (1) to 20 mg/mL. To this solution (43.75 mL) were added physiological saline containing 11.2% magnesium chloride (0.03125 mL), physiological saline containing 10 w/v% mannitol (17.45625 mL), and physiological saline (78.7625 mL), and the mixture was stirred.

### [Preparation Example 48]

To the Compound (1) was added 0.06 mol/L hydrochloric acid to dissolve the Compound (1), and 0.06 mol/L hydrochloric acid was further added to adjust the pH to 3.5. Water for injection was added to this solution to adjust the concentration of the Compound (1) to 20 mg/mL. To this solution (43.75 mL) were added physiological saline containing 11.2% magnesium chloride (0.0625 mL), physiological saline containing 10 w/v% mannitol (17.36875 mL), and physiological saline (78.81875 mL), and the mixture was stirred.

### [Preparation Example 49]

To the Compound (1) was added 0.06 mol/L hydrochloric acid to dissolve the Compound (1), and 0.06 mol/L hydrochloric acid was further added to adjust the pH to 3.5. Water for injection was added to this solution to adjust the concentration of the Compound (1) to 20 mg/mL. To this solution (43.75 mL) were added physiological saline containing 11.2% magnesium chloride (0.125 mL), physiological saline containing 10 w/v% mannitol (17.2375 mL), and physiological saline (78.8875 mL), and the mixture was stirred.

### [Preparation Example 50]

To the Compound (1) was added 0.06 mol/L hydrochloric acid to dissolve the Compound (1), and 0.06 mol/L hydrochloric acid was further added to adjust the pH to 3.5. Water for injection was added to this solution to adjust the concentration of the Compound (1) to 20 mg/mL. To this solution (43.75 mL) were added physiological saline containing 11.2% magnesium chloride (0.25 mL), physiological saline containing 10 w/v% mannitol (16.93125 mL), and physiological saline (79.06875 mL), and the mixture was stirred.

### [Preparation Example 51]

To the Compound (1) was added 0.06 mol/L hydrochloric acid to dissolve the Compound (1), and 0.06 mol/L hydrochloric acid was further added to adjust the pH to 3.5. Water for injection was added to this solution to adjust the concentration of the Compound (1) to 20 mg/mL. To this solution (43.75 mL) were added physiological saline containing 11.2% magnesium chloride (0.5 mL), physiological saline containing 10 w/v% mannitol (16.31875 mL), and physiological saline (79.43125 mL), and the mixture was stirred.

### [Test Example 6] (Repeated intravenous administration test in rat)

The pharmaceutical solutions of Preparation Examples 47, 48, 49, 50, and 51, the pharmaceutical solution of Contrast Example 9, and physiological saline were respectively administered to rats repeatedly from their tail vein once a day and 14 times in total (dosing volume, 10 mL/kg/day; administration rate, 1 mL/min; 5 animals per group). The animals were sacrificed and the administration site was observed visually and subjected to histological test (5 samples were collected from the vein which is the administration site per animal, and the tissue finding was scored. The larger score means the stronger toxicity). The results are shown in Table 4. The score of the histological test shown is the average of the scores.
As demonstrated in Table 4, while swelling (4 cases in 5 cases) and erythema (1 case in 5 cases) were confirmed at the site of administration in the case of Contrast Example (Contrast Example 9), such symptoms were not at all observed in the case of the pharmaceutical solution of the present invention having 0.05 mg/mL or more (0.016 or more in molar ratio to the Compound (1)) of magnesium chloride added thereto (Preparation Examples 48, 49, 50, and 51). The pharmaceutical solutions of the present invention also showed scores lower than those of Contrast Examples in the necrosis and degeneration of the vascular wall and all tissue findings. In particular, necrosis and degeneration of the vascular wall was not at all noted when the pharmaceutical solution of the present invention having 0.4 mg/mL (at a molar ratio of 0.13 in relation to the Compound (1)) of magnesium chloride added thereto was used.

**Table 4**

| The results of local irritation evaluation (Test Example 6) | | | | | | |
|---|---|---|---|---|---|---|
| Concentration of Compound (1) (mg/mL) | Concent ration of MgCl₂ (mg/mL) | Molar ratio of MgCl₂/ Compound (1) | Dosing volume | Result of visual observation | Result of histological test Score-1^{*1} | Result of histological test Score-2^{*2} |
| 0 | 0 | - | Physiological saline | No change | 0 | 4.32 |
| 6.25 | 0 | 0 | Contrast Ex. (9) | Erythema (1 in 5) swelling (4 in 5) | 1.00 | 10.80 |
| 6.25 | 0.025 | 0.008 | Prep. Ex.(47) | Crust formation (1 in 5) swelling (5 in 5) | 0.64 | 9.40 |
| 6.25 | 0.05 | 0.016 | Prep. Ex. (48) | No change | 0.32 | 8.00 |
| 6.25 | 0.1 | 0.031 | Prep. Ex.(49) | No change | 0.52 | 8.32 |
| 6.25 | 0.2 | 0.063 | Prep. Ex. (50) | No change | 0.40 | 6.68 |
| 6.25 | 0.4 | 0.13 | Prep. Ex. (51) | No change | 0.00 | 5.72 |

| | | | | | | |
|---|---|---|---|---|---|---|
| *1 Score-1: Average score of necrosis and degenration of vascular wall *2 Score-2: Average score of all tissue finfings | | | | | | |

### [Contrast Example 10]

(1) To the Compound (1) (8.76 or 8.77 g) was added 0.06 mol/L hydrochloric acid to dissolve the Compound (1), and 0.06 mol/L hydrochloric acid was further added to adjust the pH to 3.5. Water for injection was added to this solution to a final volume of 420 mL. To this solution (132.25 mL) were added water for injection (5.75 mL), physiological saline containing 10 w/v% mannitol (55.2 mL), and physiological saline (248.4 mL), and the mixture was stirred.
(2) To the Compound (1) (7.30 g) was added 0.06 mol/L hydrochloric acid to dissolve the Compound (1), and 0.06 mol/L hydrochloric acid was further added to adjust the pH to 3.5. Water for injection was added to this solution to a final volume of 350 mL. To this solution (109.25 mL) were added water for injection (4.75 mL), physiological saline containing 10 w/v% mannitol (45.6 mL), and physiological saline (205.2 mL), and the mixture was stirred.

### [Contrast Example 11]

The solution of the Contrast Example 10 was mixed with an equal amount of physiological saline.

### [Preparation Example 52]

(1) To the Compound (1) (8.76 g or 8.77 g) was added 0.06 mol/L hydrochloric acid to dissolve the Compound (1), and 0.06 mol/L hydrochloric acid was further added to adjust the pH to 3.5. Water for injection was added to this solution to a final volume of 420 mL. To this solution (138 mL) were added water for injection (4.5 mL), 12.72% aqueous solution of magnesium chloride (1.5 mL), physiological saline containing 10 w/v% mannitol (53.28 mL), and physiological saline (263.52 mL), and the mixture was stirred.
(2) To the Compound (1) (7.30 g) was added 0.06 mol/L hydrochloric acid to dissolve the Compound (1), and 0.06 mol/L hydrochloric acid was further added to adjust the pH to 3.5. Water for injection was added to this solution to a final volume of 350 mL. To this solution (115 mL) were added water for injection (3.75 mL), 12.72% aqueous solution of magnesium chloride (1.25 mL), physiological saline containing 10 w/v% mannitol (44.4 mL), and physiological saline (219.6 mL), and the mixture was stirred.

### [Preparation Example 53]

The solution of the Preparation Example 52 was mixed with an equal amount (160 mL) of physiological saline.

### [Preparation Example 54]

(1) To the Compound (1) (8.76 g or 8.77 g) was added 0.06 mol/L hydrochloric acid to dissolve the Compound (1), and 0.06 mol/L hydrochloric acid was further added to adjust the pH to 3.5. Water for injection was added to this solution to a final volume of 420 mL. To this solution (138 mL) were added 12.72% aqueous solution of magnesium chloride (6 mL), physiological saline containing 10 w/v% mannitol (43.2 mL), and physiological saline (273.6 mL), and the mixture was stirred.
(2) To the Compound (1) (7.30 g) was added 0.06 mol/L hydrochloric acid to dissolve the Compound (1), and 0.06 mol/L hydrochloric acid was further added to adjust the pH to 3.5. Water for injection was added to this solution to a final volume of 350 mL. To this solution (115 mL) were added 12.72% aqueous solution of magnesium chloride (5 mL), physiological saline containing 10 w/v% mannitol (36.0 mL), and physiological saline (228.0 mL), and the mixture was stirred.

### [Preparation Example 55]

The solution of the Preparation Example 54 was mixed with an equal amount (160 mL) of physiological saline.

### [Test Example 7] (Repeated 4-week intravenous administration test in monkey)

The pharmaceutical solutions of Preparation Examples 52, 53, 54, and 55, the pharmaceutical solutions of Contrast Examples 10 and 11, and aqueous solution of magnesium chloride (magnesium chloride concentration, 1.66 mg/mL; pH 4) were respectively administered to monkeys repeatedly from their cephalic vein or in the saphenous vein once a day, and 28 times in total (dosing volume, 10 mL/kg/day; administration rate, 5 mL/min; 2 animals per group). The animals were sacrificed and histological test of the administration site was conducted (samples were collected from left and right, fore- and hind-limbs at 2 sites per limb, namely, 8 samples per animal, and the tissue finding was scored. The larger score means the severe irritation). The results are shown in Table 5. The score of the histological test shown is the average of the scores.
As demonstrated in Table 5, when the pharmaceutical solution was used at a high concentration (concentration of the Compound (1), 6.25 mg/mL), the pharmaceutical solutions of the present invention having magnesium chloride added thereto (Preparation Examples 52 and 54) showed lower scores compared to the Contrast Example (Contrast Example 10) in the necrosis and degeneration of the vascular wall. The pharmaceutical solutions of the present invention also showed scores lower than those of Contrast Examples in all tissue findings irrespective of the concentration of the Compound (1).

**Table 5**

| The results of local irritation evaluation (Test Example 7) | | | | | |
|---|---|---|---|---|---|
| Concentration of Compound (1) (mg/mL) | Concentration of MgCl₂ (mg/mL) | Molar ratio of MgCl₂/ Compound (1) | Dosing solution | Result of histological test Score-1^{*1} | Result of histological test Score-2^{*2} |
| 3.125 | 0 | 0 | Contrast Ex.(11) | 0 | 2.14 |
| 3.125 | 0.21 | 0.13 | Prep. Ex.(53) | 0 | 1.19 |
| 3.125 | 0.83 | 0.53 | Prep. Ex.(55) | 0 | 1.50 |
| 6.25 | 0 | 0 | Contrast Ex.(10) | 0.44 | 5.31 |
| 6.25 | 0.41 | 0.13 | Prep. Ex.(52) | 0 | 3.25 |
| 6.25 | 1.66 | 0.53 | Prep. Ex.(54) | 0 | 1.63 |
| 0 | 1.66 | - | MgCl₂ aqueous solution, pH 4 | 0 | 1.38 |

| | | | | | |
|---|---|---|---|---|---|
| *1 Score-1: Average score of necrosis and degeneration of vascular wall *2 Score-2: Average score of all tissue findings | | | | | |

### [Contrast Example 12]

(1) To the Compound (1) (9.6 g) was added 0.06 mol/L hydrochloric acid to dissolve the Compound (1), and 0.06 mol/L hydrochloric acid was further added to adjust the pH to 3.5. Water for injection was added to this solution to a final volume of 480 mL. To this solution (50 mL) were added physiological saline containing 10 w/v% mannitol (20 mL) and physiological saline (90 mL), and the mixture was stirred.
(2) To the Compound (1) (6.8 g) was added 0.06 mol/L hydrochloric acid to dissolve the Compound (1), and 0.06 mol/L hydrochloric acid was further added to adjust the pH to 3.5. Water for injection was added to this solution to a final volume of 340 mL. To this solution (50 mL) were added physiological saline containing 10 w/v% mannitol (20 mL) and physiological saline (90 mL), and the mixture was stirred.

### [Contrast Example 13]

(1) To the Compound (1) (9.6 g) was added 0.06 mol/L hydrochloric acid to dissolve the Compound (1), and 0.06 mol/L hydrochloric acid was further added to adjust the pH to 3.5. Water for injection was added to this solution to a final volume of 480 mL. To this solution (50 mL) were added physiological saline containing 10 w/v% sorbitol (20 mL) and physiological saline (90 mL), and the mixture was stirred.
(2) To the Compound (1) (6.8 g) was added 0.06 mol/L hydrochloric acid to dissolve the Compound (1), and 0.06 mol/L hydrochloric acid was further added to adjust the pH to 3.5. Water for injection was added to this solution to a final volume of 340 mL. To this solution (50 mL) were added physiological saline 10 w/v% sorbitol (20 mL) and physiological saline (90 mL), and the mixture was stirred.

### [Preparation Example 56]

(1) To the Compound (1) (9.6 g) was added 0.06 mol/L hydrochloric acid to dissolve the Compound (1), and 0.06 mol/L hydrochloric acid was further added to adjust the pH to 3.5. Water for injection was added to this solution to a final volume of 480 mL. To this solution (218.75 mL) were added 2.5% aqueous solution of magnesium chloride (11.2 mL), physiological saline containing 10 w/v% mannitol (81.594 mL) and physiological saline (388.456 mL), and the mixture was stirred.
(2) To the Compound (1) (6.8 g) was added 0.06 mol/L hydrochloric acid to dissolve the Compound (1), and 0.06 mol/L hydrochloric acid was further added to adjust the pH to 3.5. Water for injection was added to this solution to a final volume of 340 mL. To this solution (78.125 mL) were added 2.5% aqueous solution of magnesium chloride (4 mL), physiological saline containing 10 w/v% mannitol (29.141 mL) and physiological saline (138.734 mL), and the mixture was stirred.

### [Preparation Example 57]

(1) To the Compound (1) (9.6 g) was added 0.06 mol/L hydrochloric acid to dissolve the Compound (1), and 0.06 mol/L hydrochloric acid was further added to adjust the pH to 3.5. Water for injection was added to this solution to a final volume of 480 mL. To this solution (50 mL) were added 2.5% aqueous solution of magnesium chloride (2.56 mL), physiological saline containing 10 w/v% sorbitol (18.65 mL) and physiological saline (88.79 mL), and the mixture was stirred.
(2) To the Compound (1) (6.8 g) was added 0.06 mol/L hydrochloric acid to dissolve the Compound (1), and 0.06 mol/L hydrochloric acid was further added to adjust the pH to 3.5. Water for injection was added to this solution to a final volume of 340 mL. To this solution (50 mL) were added 2.5% aqueous solution of magnesium chloride (2.56 mL), physiological saline containing 10 w/v% sorbitol (18.65 mL) and physiological saline (88.79 mL), and the mixture was stirred.

### [Test Example 8] (Repeated intravenous administration test in rat)

The pharmaceutical solutions of Preparation Examples 56 and 57, the pharmaceutical solutions of Contrast Examples 12 and 13, and physiological saline adjusted to pH 4 were respectively administered to rats repeatedly from their tail vein once a day and 14 times in total (dosing volume, 10 mL/kg/day; administration rate, 1 mL/min; 5 animals per group). The animals were sacrificed and the administration site was subjected to autopsy and histological test (5 samples were collected from the vein which is the administration site per animal, and the tissue deformation was scored. The larger score means the severe irritation). The results are shown in Tables 6 and 7. The score of the histological test shown is the average of the scores.
As demonstrated in Table 6, while leukoderma, erythema, swelling, crust formation, and erosion were confirmed at the site of administration in the case of Contrast Examples (Contrast Examples 12 and 13) irrespective of the type of the isotonic agent used, such symptoms were not observed in the case of the pharmaceutical solutions of the present invention having magnesium chloride added thereto (Preparation Examples 55 and 56) except for the swelling confirmed in 1 case out of 5 cases when sorbitol was used for the isotonic agent.
In addition, as demonstrated in Table 7, the pharmaceutical solutions of the present invention also showed scores lower than those of Contrast Examples in the necrosis and deformation of the vascular wall and all tissue deformations irrespective of the type of the isotonic agent used.

**Table 6**

| The results of local irritation evaluation (Test Example 8) | | | | | |
|---|---|---|---|---|---|
| Concentration of Compound (1) (mg/mL) | Concent ration of MgCl₂ (mg/mL) | Molar ratio of MgCl₂/ Compound (1) | Isotonic agent | Dosing solution | Results of the autopsy |
| 0 | 0 | 0 | - | Physiolo gical saline, pH 4 | No change |
| 6.25 | 0 | 0 | Mannitol | Contrast | Leukoderma (1 in 5) |
| | | | | Ex.(12) | erythema (1 in 5) swelling (5 in 5) crust formation (5 in 5) erosion (2 in 5) |
| 6.25 | 0.4 | 0.13 | Mannitol | Prep. Ex.(56) | No change |
| 6.25 | 0 | 0 | Sorbitol | Contrast Ex. (13) | Leukoderma (1 in 5) erythema (1 in 5) swelling (4 in 5) crust formation (1 in 5) erosion (1 in 5) |
| 6.25 | 0.4 | 0.13 | Sorbitol | Prep. Ex.(57) | swelling (1 in 5) |

**Table 7**

| The results of local irritation evaluation (Test Example 8) | | | | | | |
|---|---|---|---|---|---|---|
| Concen tration of Compou nd (1) (mg/mL ) | Concent ration of MgCl₂ (mg/mL) | Molar ratio of MgCl₂/ Compound (1) | Isotonic agent | Solution administered_ | Result of histological test Score-1^{*1} | Result of histological test Score-2^{*2} |
| 0 | 0 | 0 | - | Physiological saline, pH 4 | 0 | 4.12 |
| 6.25 | 0 | 0 | Mannitol | Contrast Ex. (12) | 0.60 | 12.32 |
| 6.25 | 0.4 | 0.13 | Mannitol | Prep. Ex.(56) | 0.16 | 4.84 |
| 6.25 | 0 | 0 | Sorbitol | Contrast Ex. (13) | 0.65 | 8.50 |
| 6.25 | 0.4 | 0.13 | Sorbitol | Prep. Ex.(57) | 0.20 | 4.44 |

| | | | | | | |
|---|---|---|---|---|---|---|
| *1 Score-1: Average score of necrosis and degeneration of vascular wall *2 Score-2: Average score of all tissue findings | | | | | | |

Formulation of the lyophilized product containing Compound (1), and in particular, formulation adapted for preparing a lyophilized product which is free from multivalent metal that can be combined with the multivalent metal-containing reconstitution solution was investigated.

### [Lyophilized product 1]

To water for injection (800 mL) was added 1 mol/L hydrochloric acid (50 mL). To this aqueous solution was added the Compound (1) (20 g), and then, 0.1 mol/L hydrochloric acid to adjust the pH to 3.5. Water for injection was added to this solution to adjust the content of the Compound (1) to 20 mg/mL. This solution was filled in containers at 10 mL per container, lyophilized by the procedure as described below, and tightly sealed.
i) The container having the solution of the Compound (1) filled therein is stored in the shelf of a lyophilizer that has been adjusted to 5°C.
ii) The shelf temperature is continuously cooled to -40°C at a cooling rate of 0.1°C/minutes.
iii) The shelf temperature is kept at -40°C for at least 3 hours.
iv) Treatment at reduced pressure is started, and the shelf temperature is set at -5°C and this temperature is maintained for at least 30 hours.
v) When the temperature of the article reaches -5°C or higher, the shelf temperature is set at 25°C and this temperature is maintained for at least 6 hours. During this step, a reduced pressure of 1 Pa is kept.
   To the lyophilized product of the present invention, 10 mL of water for injection was added for reconstitution. 236 seconds was required for the reconstitution.

**Table 8**

| Conditions used in producing the lyophilized product | | | | | | | |
|---|---|---|---|---|---|---|---|
| Formulation | Proportion of Compound (1) (mg/mL) | Amount of solution filled (mL) | Cooling rate (°C/min) | Primary drying | | Secondary drying | |
| | | | | Temp. (°C) | Time (hr) | Temp. (°C) | Time (hr) |
| Lyophilized product 1 | 20-0 | 10 | 0.1 | -5 | at least 30 | 25 | at least 6 |

### [Detection of related substance and new related substance]

The thus produced lyophilized products were reconstituted to determine whether related substance or new related substance had generated in the reconstituted solution, and the amount of such substance was also measured when such substance had generated. A related substance is an impurity such as the decomposition product of the Compound (1), and the new related substance is an impurity formed by the reaction of the analogous substance with the additive.
The related substance and the new related substance were measured for 10 µL of the standard solution and the sample solution under the HPLC conditions as described below. The results are shown in Table 9.

### [Standard Solution]

About 20 mg of the standard sample of the Compound (1) was weighed and dissolved with mobile phase B so that the mixture was accurately 50 mL. This solution was filtered through 0.45 µm filter, and after discarding the initial 5 mL filtrate, the subsequent filtrate was used as the standard solution.

### [Sample solution]

One lyophilized product was reconstituted by adding the same amount of water for injection as that of drug solution filled, and the solution was transferred to a 100 mL volumetric flask. Mobile phase B was added to the solution accurately to a volume of 100 mL. 10 mL of this solution was accurately measured, and mobile phase B was added to the solution accurately to the total volume of 50 mL. This solution was filtered through 0.45 µm filter, and after discarding the initial 5 mL filtrate, the subsequent filtrate was used as the sample solution.

### [10mM phosphate buffer]

7.16 g of disodium hydrogen phosphate dodecahaydrate and 3.12 g of sodium dihydrogen phosphate dihydrate were measured, and dissolved in 4 L of water. This solution was filtered through a 0.45 µm filter.

### [Mobile phase A]

3 L of 10mM phosphate buffer was mixed with 1 L of acetonitrile.

### [Mobile phase B]

1 L of 10mM phosphate buffer was mixed with 3 L of acetonitrile.

### [HPLC conditions]

Detector: UV absorptiometer (wavelength, 292 nm)
Column: Symmetry C18
Column temperature: 40°C
Mobile phase: mobile phase A, mobile phase B, and their gradient
Flow rate: about 1.0 mL/min
Injection volume: 10 µL
Measurement time: 30 min
Washing solution of the autosampler: water/acetonitrile (3:7)

**Table 9**

| Formulation | Proportion of Compound (1) (mg/mL) | Insoluble particlulate matter¹⁾ 60°C, 2 W | New related substance | Total amount of related substance (Area%) | |
|---|---|---|---|---|---|
| | | | | Initial | 60°C, 2 W |
| Lyophilized product 1 | 20 | - | None | 0.85 | 1.32 |
| | | | | | |
| 1) Insoluble particulate matter | | -: None | | | |
| | | ±: less than 10 / container (50 µm to 100 µm) | | | |
| | | +: 10 or more / container (50 µm to 100 µm) | | | |

As demonstrated in Table 9, with regard to the lyophilized product containing the Compound (1), a preparation exhibiting favorable properties can be produced by using the formulation comprising the Compound (1) and an adequate amount of pH adjusting agent.

### [Evaluation of the concentration of the drug solution used for the lyophilization]

According to the example of the formulation of the lyophilized product 1, drug solutions containing different amounts of the Compound (1) were produced, and the solutions were lyophilized to determine the adequate concentration of the Compound (1) in the drug solution used for producing the lyophilized product. The results are shown in Table 10. As demonstrated in this Table, concentration of the Compound (1) in the drug solution affects the degree of cake formation of the lyophilized product.
More specifically, good cake formation of the lyophilized product was found to be realized when the concentration of the Compound (1) in the drug solution was 10 mg/mL or higher.

**Table 10**

| Evaluation of the concentration of the lyophilized product of Compound (1) for the cake formation | |
|---|---|
| Concentration of the drug solution containing the Compound (1) (mg/mL) | Cake formation¹⁾ |
| 20 mg/mL | 5/5 |
| 15 mg/mL | 5/5 |
| 10 mg/mL | 5/5 |
| 5 mg/mL | 4/5 |
| 2.5 mg/mL | 4/5 |
| 1.25 mg/mL | 0/5 |

| | |
|---|---|
| 1) Number of vials with cake formation / number of vials tested | |

Accordingly, the drug solution containing the Compound (1) used for preparing the lyophilized product of Compound (1) should contain the Compound (1) at a concentration of at least about 5 mg/mL, preferably at least 10 mg/mL, more preferably at least 15 mg/mL, and most preferably at least 20 mg/mL.

### [Evaluation of the pH of the drug solution used for the lyophilization (1)]

Change in solubility with the change in pH of the drug solution of the Compound (1) was measured by the method commonly used in the art. The results are shown in Table 11.

**Table 11**

| Solubility of Compound (1) in water | | |
|---|---|---|
| | Immediately after preparation | After 1 W |
| pH | | Solubility |
| | 25°C | |
| pH 3.5 | ≥ 20.1 mg/mL | ≥ 20.1 mg/mL |
| pH 4.0 | ≥ 20.1 mg/mL | 19.6 mg/mL |
| pH 4.5 | ≥ 20.1 mg/mL | 20.1 mg/mL |
| pH 5.0 | - | 18.7 mg/mL |
| pH 5.5 | - | 4.8 mg/mL |
| pH 6.0 | - | 0.7 mg/mL |
| 5°C | | |
| pH 3.5 | ≥ 20.1 mg/mL | ≥ 19.6 mg/mL |
| pH 4.0 | ≥ 20.1 mg/mL | 19.0 mg/mL |
| pH 4.5 | ≥ 20.0 mg/mL | 18.8 mg/mL |
| pH 5.0 | - | 18.4 mg/mL |
| pH 5.5 | - | 5.4 mg/mL |
| pH 6.0 | - | 0.7 mg/mL |

As demonstrated in Table 11, a pharmaceutical solution having a concentration sufficient for the cake formation of the lyophilized product can be obtained when the solution has a pH of up to about 5. More specifically, the concentration of the Compound (1) should preferably be at least 10 mg/mL in view of the cake formation, and this concentration can be attained when the pH is up to pH 5. It was also revealed that a concentration of 20 mg/mL or more can be attained when the pH is up to 4.5.
Accordingly, the pharmaceutical solution should have a pH of up to 5.5, preferably up to 5, and more preferably in the range of 4.5 to 2.5 in view of preparing the lyophilized product of the Compound (1).

### [Evaluation of pH of the drug solution used for the lyophilization (2)]

Lyophilized products were produced from the drug solution of different pH according to the evaluation of pH of the drug solution used for the lyophilization (1) to evaluate the stability of the Compound (1). The results are shown in Table 12.

**Table 12**

| Results of the stability test of the lyophilized product | | | | | |
|---|---|---|---|---|---|
| Sample | Content in relation to | | Total amount of related substance (%) | pH | Appearance |
| | Nominal amount (%) | Initial amount (%) | | | |
| pH 2.5, Initial | 95.3 | 100.0 | 0.92 | 2.89 | Yellow |
| pH 2.5, 60°C, 1 W | 96.0 | 100.8 | 0.95 | 2.97 | Yellow |
| pH 2.5, 60°C, 2 W | 91.9 | 96.5 | 1.10 | 3.05 | Yellow |
| pH 3.5, Initial | 96.5 | 100.0 | 0.93 | 3.73 | Yellow |
| pH 3.5, 60°C, 1 W | 96.0 | 99.4 | 0.96 | 3.81 | Yellow |
| pH 3.5, 60°C, 2 W | 96.2 | 99.7 | 1.17 | 3.86 | Yellow |
| pH 4.5, Initial | 93.1 | 100.0 | 0.93 | 4.47 | Yellow |
| pH 4.5, 60°C, 1 W | 91.1 | 97.8 | 1.13 | 4.51 | Yellow |
| pH 4.5, 60°C, 2 W | 93.5 | 100.5 | 1.31 | 4.53 | Yellow |

As demonstrated in Table 12, the Compound (1) showed sufficient stability in the lyophilized products produced from the drug solution containing the Compound (1) at a pH in the range of 2.5 to 4.5.

### [Exemplary formulation of the lyophilized product of the Compound (1)]

Based on the investigation as described above, an exemplary formulation of the lyophilized product of the Compound (1) is shown in Table 13.

**Table 13**

| Formulation of the lyophilized product | | |
|---|---|---|
| Function | Ingredient | Content in 1 vial (in 10 mL) |
| Active ingredient | Compound (1) | 200 mg |
| pH adjusting agent | Hydrochloric acid | Appropriate amount (pH 2.5 to 4.5) |
| pH adjusting agent | Sodium hydroxide | |
| (Solvent) | (water for injection) | (Total volume, 10 mL) |

## Claims

1. A pharmaceutical solution comprising the following components (A) and (B):
(A) a compound represented by the following formula (1): 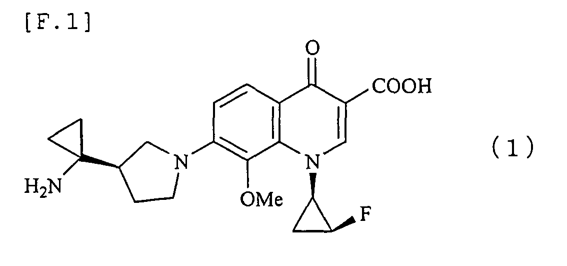 or a salt thereof, or a hydrate thereof; and
(B) a multivalent metal compound, wherein molar ratio of component (B) to component (A) (the multivalent compound / component (A)) is 0.01 to 0.7.

2. The pharmaceutical solution according to claim 1, wherein the multivalent metal of the multivalent metal compound is the one capable of forming a metal complex with component (A).

3. The pharmaceutical solution according to claim 1 or 2, wherein the multivalent metal compound is a magnesium compound or a calcium compound.

4. The pharmaceutical solution according to claim 3, wherein the multivalent metal compound is a magnesium compound.

5. The pharmaceutical solution according to claim 4, wherein the magnesium compound is magnesium chloride.

6. The pharmaceutical solution according to any one of claims 1 to 5, wherein pH is in the range of 2.5 to 6.9.

7. The pharmaceutical solution according to any one of claims 1 to 6, wherein the pharmaceutical solution is adapted for intravascular administration.

8. A preparation for preparing the pharmaceutical solution of any one of claims 1 to 7 containing the component (A) and the multivalent metal compound.

9. A set for preparing the pharmaceutical solution of any one of claims 1 to 7 comprising a combination of a preparation containing the component (A), and a reconstitution solution containing the multivalent metal compound.

10. A set for preparing the pharmaceutical solution of any one of claims 1 to 7 comprising a combination of a preparation containing the component (A) and the multivalent metal compound, and a reconstitution solution.

11. The preparation or the set according to any one of claims 8 to 10, wherein the preparation containing the component (A) further comprises a pharmaceutical additive.

12. The preparation or the set according to any one of claims 8 to 11, wherein the preparation containing the component (A) is a lyophilized product.

13. The preparation or the set according to any one of claims 8 to 12 further comprising a diluent which optionally contains the multivalent metal compound.

14. A set for preparing the pharmaceutical solution of any one of claims 1 to 7 comprising a combination of a solution preparation containing the component (A) and a solution preparation containing the multivalent metal compound.

15. The set according to claim 14, which further comprises a diluent optionally containing the multivalent metal compound.

16. A method for alleviating the toxicity developed at the site to be administered with component (A), **characterized in that** the administration of compound (A) represented by the following formula (1): 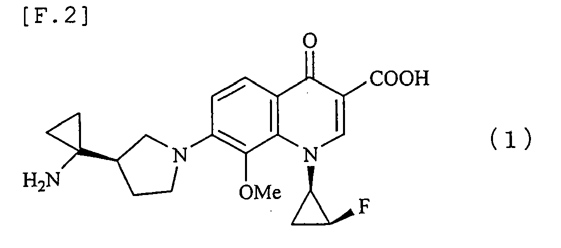 or a salt thereof, or a hydrate thereof, is carried out under the conditions where the multivalent metal compound is present at a molar ratio to compound (A) (the multivalent metal / component (A)) of 0.01 to 0.7 in the pharmaceutical solution containing the component (A).

17. The method according to claim 16, wherein the multivalent metal compound is incorporated in the pharmaceutical solution by adding a reconstitution solution and/or the diluent (one or both of the reconstitution solution and the diluent containing the multivalent metal compound) to the solution preparation and/or the preparation containing component (A).

18. The method according to claim 16 or 17, wherein the multivalent metal compound is dissolved in the solution preparation containing component (A).

19. The method according to any one of claims 16 to 18, wherein the multivalent metal compound is a magnesium compound or a calcium compound.

20. The method according to claim 19, wherein the multivalent metal compound is a magnesium compound.

21. The method according to claim 20 to 20, wherein the magnesium compound is magnesium chloride.

22. The method according to any one of claims 16 to 21, wherein the pharmaceutical solution containing the component (A) having the multivalent metal compound incorporated therein has a pH in the range of 2.5 to 6.9.

23. The method according to any one of claims 16 to 22, wherein the site of the administration is blood vessel.
